# EUROPEAN PATENT APPLICATION

(11) **EP 1 510 192 A2**
(43) Date of publication of application: **02.03.2005**
(21) Application number: 04255209.1
(22) Date of filing: 27.08.2004
(51) Int. Cl.: A61F 13/511, A61F 13/514, B32B 3/28, B31F 1/07

(54) **Two layer structure for absorbent articles**

(30) Priority: 29.08.2003 US 651676
(71) Applicant: McNeil-PPC, Inc., Skillman, New Jersey 08558 (US)
(72) Inventor: Kelly, William G. F., Middlesex, NJ 08846 (US); James, William A., Hopewell, NJ 08525 (US); Jones, Archie, Somerset, NJ 08873 (US)
(74) Representative: Mercer, Christopher Paul

(57) **Abstract**

A two layer structure comprising a fluid permeable, first layer in fluid communication with a fluid permeable second layer is provided. The two layers contact one another through a plurality of disconnected macrofeatures that project either from the first layer or the second layer. The structure has particular utility as a cover/transfer layer for use in absorbent articles.

## Description

### FIELD OF THE INVENTION

This present invention relates to a two-layer structure for use in absorbent articles, and more particularly to a two-layer structure including a fluid permeable first layer in fluid communication with a fluid permeable second layer, the second layer including a plurality of disconnected macrofeatures. The structure is particularly useful as a cover/transfer layer for use in absorbent articles.

### BACKGROUND OF THE INVENTION

Transfer layers are commonly used in absorbent articles to aid in the transport of fluid away from a bodyfacing layer or cover towards the absorbent core. Conventional transfer layers are often made of nonwovens. They typically function by pumping or wicking fluid away from the body facing layer directly downward into the underlying absorbent core. Combination cover/transfer layers are also known. See for example, US Patent Nos. 5,665,082; 5,797,894; and 5,466,232.

Applicants have discovered that a two layer structure comprising a fluid permeable first layer in fluid communication with a fluid permeable second layer, said layers contacting one another at least at the plurality of disconnected macrofeatures, functions efficiently, among other things, as a body facing layer or cover/transfer layer. Upon insult of the first layer of this structure by a fluid, the structure moves and/or transfers the fluid both through and across the structure, thereby allowing the fluid to be transported more quickly through the structure in the z direction, i.e., through the first and second layers toward the absorbent core.

### SUMMARY OF THE INVENTION

According to one aspect of the invention, the invention provides a two layer structure for use in absorbent articles comprising a fluid permeable first layer in fluid communication with a fluid permeable second layer, wherein the layers contact one another substantially only at the plurality of spaced disconnected macrofeatures projecting from the second layer.

According to another aspect of the invention, the invention provides a two layer structure including a fluid permeable first layer in fluid communication with a fluid permeable second layer, the second layer having a plurality of spaced disconnected macrofeatures, wherein the first and second layers contact one another at said macrofeatures and at selected areas located between said macrofeatures.

According to yet another aspect of the invention, the invention provides a two layer structure for use in absorbent articles, comprising a fluid permeable first layer comprising a three dimensional apertured film in fluid communication with a fluid permeable second layer. The three dimensional film of the first layer comprises a plurality of apertures and a plurality of apertured macrofeatures projecting in the direction of the second layer, each apertured macrofeature being disconnected from other apertured macrofeatures, and wherein the first and second layers contact one another substantially only through said apertured macrofeatures.

According to yet another aspect of the invention, the invention provides a two layer structure for use in absorbent articles, comprising a fluid permeable first layer comprising a three dimensional apertured film in fluid communication with a fluid permeable second layer. The three dimensional film of the first layer comprises a plurality of apertures and a plurality of spaced apertured macrofeatures projecting in the direction of the second layer, each apertured macrofeature being disconnected from other apertured macrofeatures, and wherein the first and second layers contact one another at said apertured macrofeatures and at selected areas located between said apertured macrofeatures.

According to another aspect of the invention, the invention further provides a two layer structure for use in absorbent articles, comprising a fluid permeable, body contacting layer in fluid communication with a fluid permeable second layer. The second layer comprises a plurality of macrofeatures projecting in the direction of the body contacting layer and the macrofeatures are disconnected from one another. Additionally, the body contacting and second layers contact one another substantially only through the macrofeatures.

According to still another aspect of the invention, the invention further provides a two layer structure for use in absorbent articles, comprising a fluid permeable, body contacting layer in fluid communication with a fluid permeable second layer. The second layer comprises a plurality of spaced macrofeatures projecting in the direction of the body contacting layer, the macrofeatures being disconnected from one another. The body contacting and second layers contact one another through the macrofeatures and at selected areas located between said macrofeatures.

Finally, the invention relates to absorbent articles comprising such two layer structures.

### BRIEF DESCRIPTION OF THE DRAWINGS

Figure 1 is a photomicrograph of an embodiment of a three-dimensional film of the present invention.
Figure 1A is an illustration of a cross-section of the film of Figure 1 along line A-A.
Figure 2 is a photomicrograph of another embodiment of a three-dimensional film of the present invention.
Figure 2A is an illustration of a cross-section of the film of Figure 2 along line A-A.
Figure 2B is an illustration of a cross-section of the film of Figure 2 along line B-B.
Figure 3 is a photomicrograph of yet another embodiment of a three-dimensional film of the present invention.
Figure 3A is an illustration of a cross-section of the film of Figure 3 along line A-A.
Figure 4 is a photomicrograph of another embodiment of a three-dimensional film of the present invention.
Fig. 5 is a schematic illustration of one type of three dimensional topographical support member useful to make a film of the present invention.
Fig. 6 is a schematic illustration of an apparatus for laser sculpting a workpiece to form a three dimensional topographical support member useful to make a film of the present invention.
Fig. 7 is a schematic illustration of a computer control system for the apparatus of Figure 6.
Fig. 8 is a graphical enlargement of an example of a pattern file to raster drill a workpiece to produce a support member for apertured film.
Fig. 9 is a photomicrograph of a workpiece after it has been laser drilled using the file of Fig. 8.
Fig. 10 is a graphical representation of a file to laser sculpt a workpiece to produce the film of Figure 2.
Fig. 11 is a graphical representation of a file to laser sculpt a workpiece to produce a three dimensional topographical support member useful to make a film of this invention.
Fig. 12 is a photomicrograph of a workpiece that was laser sculpted utilizing the file of Fig. 11.
Fig. 12A is a photomicrograph of a cross section of the laser sculpted workpiece of Fig. 12.
Fig. 13 is a photomicrograph of an apertured film produced using the laser sculpted support member of Fig. 12.
Fig. 13A is another photomicrograph of an apertured film produced using the laser sculpted support member of Fig. 12.
Fig. 14 is an example of a file which may be used to produce a support member by laser modulation.
Fig. 14A is a graphical representation of a series of repeats of the file of Fig. 14.
Fig. 15 is an enlarged view of portion B of the file of Fig. 14.
Fig. 16 is a graphical enlargement of a pattern file used to create portion C of Fig. 14.
Fig. 17 is a photomicrograph of a support member produced by laser modulation using the file of Fig. 14.
Fig. 18 is a photomicrograph of a portion of the support member of Fig. 17.
Fig. 19 is a photomicrograph of a film produced by utilizing the support member of Fig. 17.
Fig. 20 is a photomicrograph of a portion of the film of Fig. 19.
Fig. 21 is a view of a support member used to make a film according to the invention in place on a film-forming apparatus.
Fig. 22 is a schematic view of an apparatus for producing an apertured film according to the present invention.
Fig. 23 is a schematic view of the circled portion of Fig. 22.
Fig. 24 is a photomicrograph of an apertured film of the prior art.
Fig. 25 is a photomicrograph of another example of an apertured film of the prior art.
Fig. 26 is a photomicrograph of another example of an apertured film of the present invention.
Fig. 27 depicts a cross-section of a two layer structure according to the invention.
Fig. 28 depicts a cross-section of an absorbent article comprising a two layer structure according to the invention.
Fig. 29 is a photomicrograph of a portion of an apertured film produced in accordance with the invention.
Fig. 30 is enlarged perspective view showing a portion of a two layer structure according to the invention with the upper layer thereof partially cut away to show the upper surface of the lower layer.
Fig. 31 is a cross-section view of an absorbent article including the two layer structure shown in Fig. 30, taken along line 31-31.
Fig. 32 is a simplified cross-sectional schematic illustration of a process for producing the two-layer structure shown in Fig. 30.

### DETAILED DESCRIPTION OF THE INVENTION

The present invention is directed to two layer structures particularly useful in personal care products. These structures may be used as body-contacting, facing or cover layers, as transfer or fluid handling layers, or as other components of personal care products. The structures of the invention have been found to exhibit improved fluid-handling properties when used in disposable absorbent articles such as, for instance, feminine sanitary protection products.

The first layer, which is in one embodiment a body contacting layer, may be made from any one of a variety of fluid permeable materials. As a body contacting layer, the first layer is preferably compliant, soft feeling, and non-irritating to a user's skin. The first layer should further exhibit good strikethrough and a reduced tendency to rewet, permitting bodily discharges to rapidly penetrate it and flow toward subsequent underlying layers, while not allowing such discharges to flow back through the body contacting layer to the skin of the user.

The first layer may be made from a wide range of materials including, but not limited to woven or knitted fabrics, nonwovens, apertured films, hydro-formed films, porous foams, reticulated foams, reticulated thermoplastic films, and thermoplastic scrims. In addition, the first layer may be constructed from a combination of one or more of the above materials, such as a composite layer of a nonwoven and apertured film.

Likewise, the second layer may also be made from a variety of fluid permeable materials including, but not limited to woven or knitted fabrics, nonwovens, apertured films, hydro-formed films, porous foams, reticulated foams, reticulated thermoplastic films, thermoplastic scrims, and combinations thereof.

Nonwovens and apertured films are preferred for use as both the first layer and the second layer. Suitable nonwovens may be made from any of a variety of fibers as known in the art. The fibers may vary in length from a quarter of an inch or less to an inch and a half or more. It is preferred that when using shorter fibers (including wood pulp fiber), the short fibers be blended with longer fibers. The fibers may be any of the well known artificial, natural or synthetic fibers, such as cotton, rayon, nylon, polyester, polyolefin, or the like. The nonwoven may be formed by any of the various techniques known in the art, such as carding, air laying, wet laying, melt-blowing, spunbonding and the like.

Apertured films are typically made from a starting film that is a thin, continuous, uninterrupted film of thermoplastic polymeric material. This film may be vapor permeable or vapor impermeable; it may be embossed or unembossed; it may be corona-discharge treated on one or both of its major surfaces or it may be free of such corona-discharge treatment; it may be treated with a surface active agent after the film is formed by coating, spraying, or printing the surface active agent onto the film, or the surface active agent may be incorporated as a blend into the thermoplastic polymeric material before the film is formed. The film may comprise any thermoplastic polymeric material including, but not limited to, polyolefins, such as high density polyethylene, linear low density polyethylene, low density polyethylene, polypropylene; copolymers of olefins and vinyl monomers, such as copolymers of ethylene and vinyl acetate or vinyl chloride; polyamides; polyesters; polyvinyl alcohol and copolymers of olefins and acrylate monomers such as copolymers of ethylene and ethyl acrylate and ethylenemethacrylate. Films comprising mixtures of two or more of such polymeric materials may also be used. The machine direction (MD) and cross direction (CD) elongation of the starting film to be apertured should be at least 100% as determined according to ASTM Test No. D-882 as performed on an Instron test apparatus with a jaw speed of 50 inches/minute (127 cm/minute). The thickness of the starting film is preferably uniform and may range from about 0.5 to about 5 mils or about 0.0005 inch (0.0013 cm) to about 0.005 inch (0.076 cm). Coextruded films can be used, as can films that have been modified, e.g., by treatment with a surface active agent. The starting film can be made by any known technique, such as casting, extrusion, or blowing.

Aperturing methods are known in the art. Typically, a starting film is placed onto the surface of a patterned support member. The film is subjected to a high fluid pressure differential while on the support member. The pressure differential of the fluid, which may be liquid or gaseous, causes the film to assume the surface pattern of the patterned support member. Portions of the film overlying apertures in the support member are ruptured by the fluid pressure differential to create an apertured film. A method of forming an apertured fibrous film is described in detail in commonly owned US 5,827,597 to James et al.

According to one aspect of the invention, the first layer and the second layer contact one another substantially only through a plurality of spaced apart, disconnected macrofeatures. By this is meant the layers are joined to one another substantially only at macrofeatures. The macrofeatures may be located on the first layer or the second layer. When the macrofeatures are located on the first layer, they project in the direction of the second layer. When the macrofeatures are located on the second layer, they project in the direction of the first layer.

According to another aspect of the invention, the first layer and the second layer contact one another at the plurality of spaced apart disconnected macrofeatures and at selected areas located between the spaced apart, disconnected macrofeatures.

As used herein, the term "macrofeature" means a surface projection visible to the normal, unaided human eye at a perpendicular distance of about 300 mm between the eye and the surface. Preferably, the macrofeatures each have a maximum dimension of at least about 0.15 mm. More preferably, the macrofeatures each have a maximum dimension of at least about 0.305 mm. Most preferably, the macrofeatures each have a maximum dimension of at least about 0.50 mm. The macrofeatures are discrete and disconnected from one another. That is, if an imaginary plane, i.e., a first plane, were lowered onto the first surface of the three-dimensional layer, it would touch the layer at the top of the macrofeatures in multiple discrete areas separated from one another. It is not necessary for each and every macrofeature to touch the imaginary plane; rather, the first plane is thus defined by the uppermost portions of the macrofeatures, that is, those parts of the macrofeatures projecting the farthest from the second surface of the layer.

Where the layer with macrofeatures comprises an apertured film, the film has a first surface, a second surface, and a caliper defined by a first plane and a second plane. The film comprises a plurality of disconnected macrofeatures and a plurality of apertures. The apertures are defined by sidewalls that originate in the film's first surface and extend generally in the direction of the film's second surface to terminate in the second plane. The first surface of the film is coincident with the first plane at the disconnected macrofeatures.

Where the layer with macrofeatures comprises a nonwoven, the nonwoven has a first surface, a second surface, and a caliper defined by a first plane and a second plane. The nonwoven further comprises a plurality of disconnected macrofeatures, wherein the first surface of the nonwoven is coincident with the first plane at the disconnected macrofeatures.

In one embodiment, the macrofeatures are arranged in a regular pattern relative to each other. Moreover, if the macrofeatures project from a layer that is an apertured film, the macrofeatures and the apertures are arranged in a regular configuration relative to each other on said layer. The apertures and macrofeatures recur at fixed or uniform intervals with respect to one another. The spatial relationship between the apertures and the macrofeatures define a geometric pattern that is consistently repeated throughout the surface area of the film. The apertures and macrofeatures are arranged in a regular, defined pattern uniformly repeated throughout the film.

The apertures and macrofeatures may be arranged so that there are more apertures than macrofeatures, although the relative arrangement of apertures and macrofeatures is regular. The exact sizes and shapes of the apertures and macrofeatures are not critical, as long as the macrofeatures are large enough to be visible to a normal unaided human eye at a distance of about 300 mm, and as long as the macrofeatures are discrete and disconnected from one another.

According to one embodiment of the invention, the first layer and the second layer contact one another substantially only though the macrofeatures. That is, the macrofeatures function much like spacers to hold the first layer away from the surface of the second layer except where they contact one another at the macrofeatures.

According to another embodiment of the invention, the first layer and the second layer contact one another at the macrofeatures and at selected areas located between the spaced macrofeatures. Within the areas defined by the contacting portions of the macrofeature, the first layer is arranged above said second layer such that it is spaced therefrom.

In yet another embodiment of the invention, the first layer comprises a nonwoven, while the second layer comprises either a nonwoven or an apertured film. The macrofeatures may be located on either the first layer or the second layer.

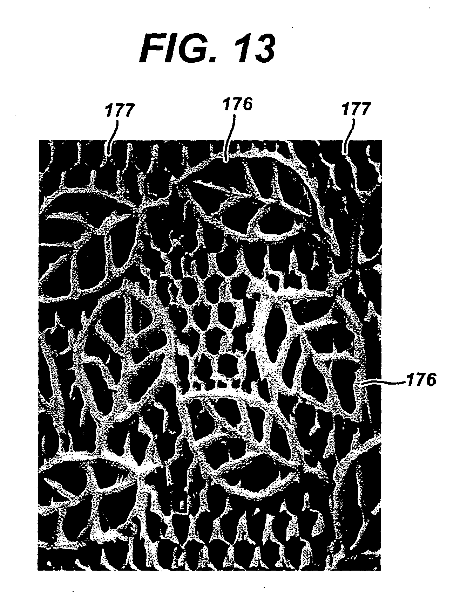

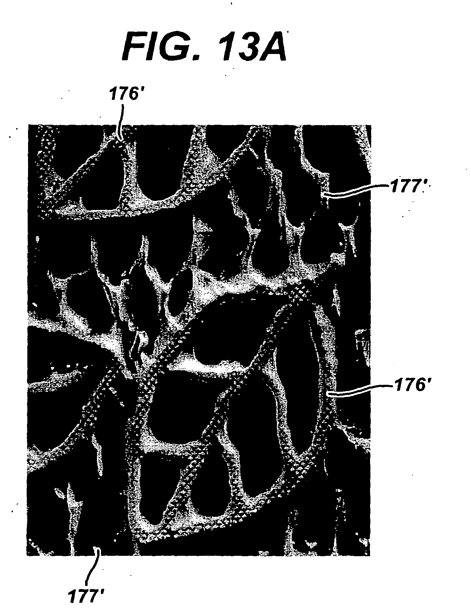

In yet another embodiment, the first layer comprises an apertured film, while the second layer comprises either a nonwoven or an apertured film. In this embodiment, the macrofeatures may also be located on either the first layer or the second layer. However, when the macrofeatures are present on the first layer, the macrofeatures on the first layer preferably contain apertures, i.e., apertured macrofeatures, and are disconnected from all other apertured macrofeatures on the first layer. Each apertured macrofeature is a discrete physical element. FIG. 13 shows a film of this embodiment, an apertured film with apertured macrofeatures.

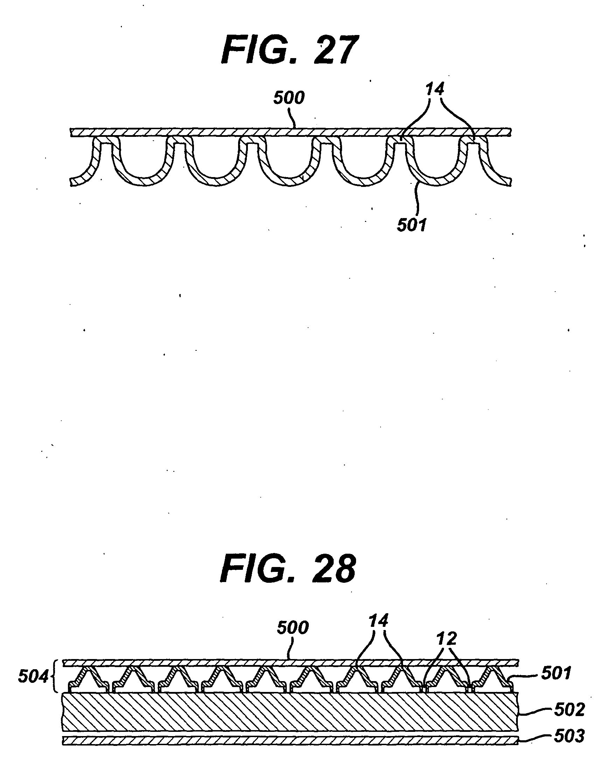

In still another embodiment of the invention, shown in FIG. 27, the macrofeatures project from the second layer, which is a three dimensional apertured film as disclosed in EP-A-1 336 399. Such a second layer 501 can be used in combination with a first layer 500 that is a nonwoven or an apertured film. Preferably, it is used in combination with a first layer that is a nonwoven. The three dimensional apertured film has a first surface and a second surface. The film additionally has a caliper defined by a first plane and a second plane. The film has a plurality of apertures defined by sidewalls that originate in the first surface and extend generally in the direction of the second surface to terminate in the second plane. The film also comprises a plurality of disconnected macrofeatures 14. The first surface of the film coincides with the first plane at these macrofeatures.

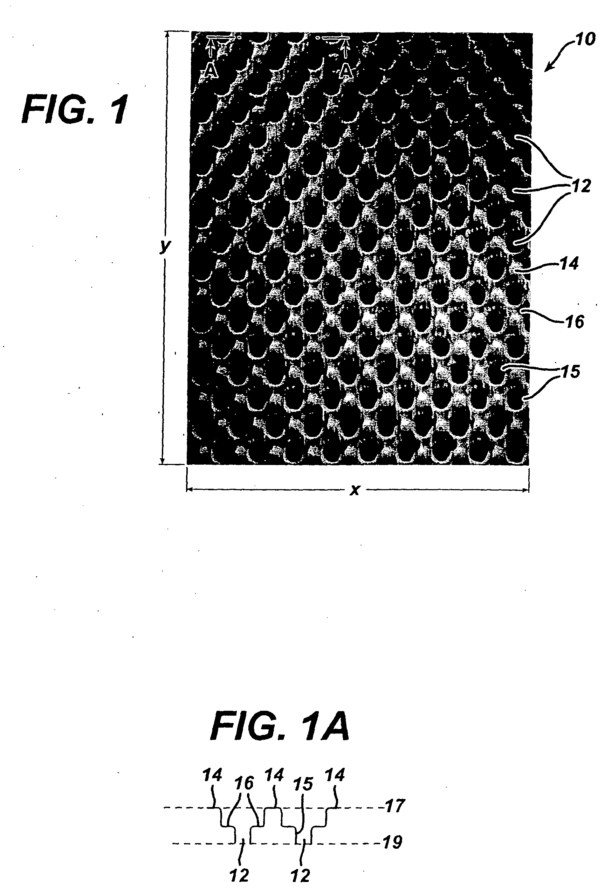

Figure 1 is a photomicrograph of an embodiment of such a three-dimensional apertured film. The film 10 of Figure 1 has apertures 12 and macrofeatures 14. The apertures are defined by sidewalls 15. The macrofeatures are discrete projections in the film and can be seen to project above lower regions 16 of the first surface. If an imaginary plane, i.e., a first plane, were lowered onto the first surface of the three-dimensional apertured film, it would touch the film at the top of the macrofeatures in multiple discrete areas separated from one another. It is not necessary for each and every macrofeature to touch the imaginary plane; rather, the first plane is thus defined by the uppermost portions of the macrofeatures, that is, those parts of the macrofeatures projecting the farthest from the second surface of the film.

In the embodiment of Figure 1, the apertures alternate with the macrofeatures in both the x-direction and the y-direction, and the ratio of apertures to macrofeatures is one.

Figure 1A is an illustration of a cross-section of the film 10 of Figure 1 along line A-A of Figure 1. As Figure 1A shows, the macrofeatures 14 are disconnected from one another in first plane 17 and are separated from one another by lower regions 16 of the first surface of the film and by apertures 12. The apertures 12 are defined by sidewalls 15 which originate in the first surface and extend generally in the direction of the second surface to terminate in second plane 19. It is not necessary for all of the apertures to terminate in the second plane 19; rather, the second plane is defined by the lowermost extending sidewalls 15.

In one embodiment of the invention, at least a portion of the apertures have sidewalls having a first portion that originates in the first plane of the film and a second portion that originates in a plane located between the first and second planes of the film, that is a plane intermediate the first and second planes.

In a preferred embodiment, in addition to having apertures with sidewalls having first portions originating in the first plane and second portions originating in an intermediate plane, the film comprises apertures whose sidewalls originate completely in an intermediate plane. That is, the film contains apertures that originate in a plane other than the plane defined by the uppermost surface of the macrofeatures.

In a particularly preferred embodiment of the present invention, the three-dimensional apertured film comprises a combination of several different types of apertures. The film comprises apertures whose sidewalls originate in the first plane of the film. The film also comprises apertures having sidewalls, a portion of which originate in the first plane and a portion of which originate in an intermediate plane. Finally, the film also comprises apertures whose sidewalls originate completely in an intermediate plane.

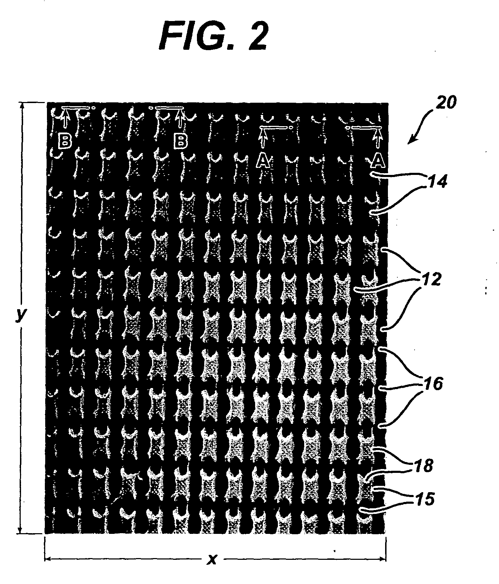

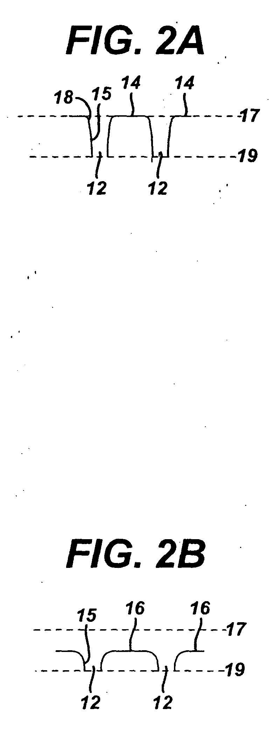

In Figure 2, apertures 12 are defined by sidewalls 15. The macrofeatures 14 project above lower regions 16 of the first surface of the film 20. The macrofeatures and apertures are shaped differently from the macrofeatures and apertures of the film of Figure 1. In Figure 2, the macrofeatures are separated from one another by apertures in the x-direction and in the y-direction. However, some of the apertures are separated from one another by lower regions 16 of the first surface in both the x-direction and the y-direction. In the film 20 of Figure 2, the ratio of apertures to macrofeatures is 2.0. Moreover, each aperture in the film 20 of Figure 2 has a portion of its sidewall originating in the first plane 17, i.e., at an edge 18 of a macrofeature, and a portion of its sidewall originating in a lower region 16 of the first surface.

Figure 2A shows a cross-section of the film 20 of Figure 2 along line A-A. The macrofeatures 14 are separated from one another in the first plane 17 by apertures 12, which are defined by sidewalls 15 that originate in the first surface of the film and extend generally in the direction of the second surface to terminate in the second plane 19. It can be seen in Figure 2A that the portions of the sidewalls 15 shown in this cross-section originate in the first plane 17 at the edges 18 of the macrofeatures 14.

Figure 2B shows a cross-section of the film 20 of Figure 2 taken along line B-B. In this particular cross-section, no macrofeatures are visible, and the apertures 12 are separated from one another by lower regions 16 of the first surface of the film. The lower regions 16 of the film lie between the first plane 17 and the second plane 19, said planes defining the caliper of the three-dimensional apertured film shown. The sidewalls 15 terminate in the second plane 19.

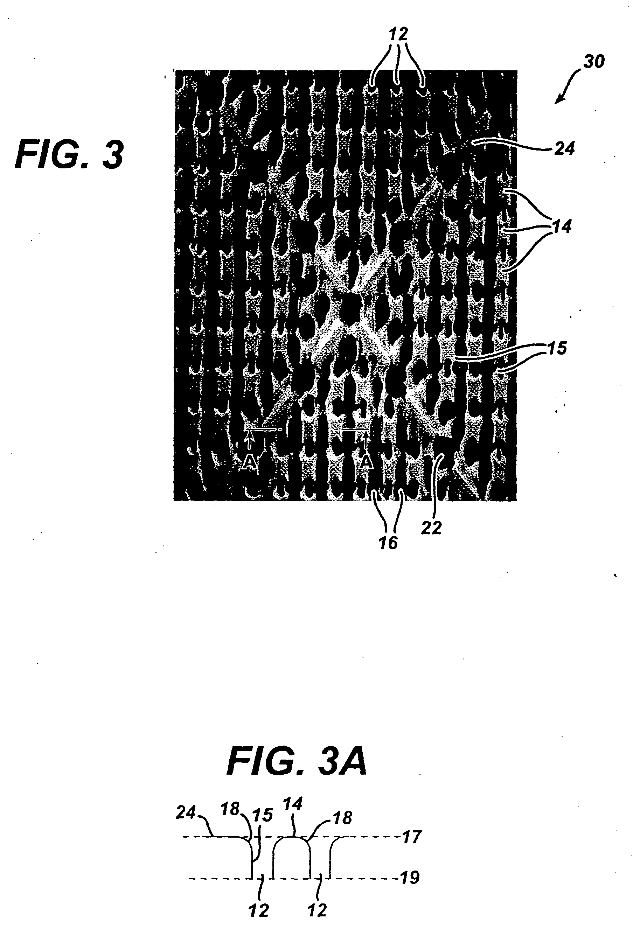

Figure 3 shows a photomicrograph of a further embodiment of a three-dimensional apertured film with yet another arrangement of apertures and macrofeatures. The film 30 of Figure 3 has apertures 12 arranged with macrofeatures 14, and apertures 22 arranged with macrofeatures 24. All of the apertures 12, 22 and macrofeatures 14, 24 are arranged together so that their relative positions to one another are regular.

Figure 3A is a cross-section of the film 30 of Figure 3 taken along line A-A of Figure 3. This particular cross-section shows macrofeatures 24 and macrofeatures 14 disconnected from one another in first plane 17 and separated from one another by apertures 12. The apertures 12 are defined by sidewalls 15 that terminate in the second plane 19. The portions of the sidewalls 15 shown in this particular cross-section originate in the first plane 17 at the edges 18 of the macrofeatures 14 and 24.

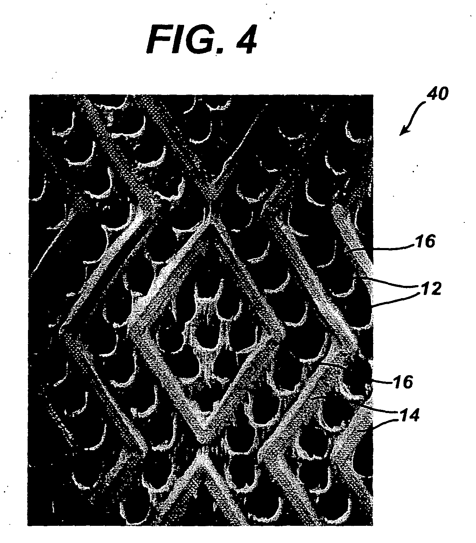

Figure 4 is a photomicrograph of yet another embodiment of a three-dimensional apertured film according to the present invention. The film 40 shown in Figure 4 has a regular arrangement of apertures 12 and macrofeatures 14.

A suitable starting film for making a three-dimensional apertured film is a thin, continuous, uninterrupted film of thermoplastic polymeric material. This film may be vapor permeable or vapor impermeable; it may be embossed or unembossed; it may be corona-discharge treated on one or both of its major surfaces or it may be free of such corona-discharge treatment; it may be treated with a surface active agent after the film is formed by coating, spraying, or printing the surface active agent onto the film, or the surface active agent may be incorporated as a blend into the thermoplastic polymeric material before the film is formed. The film may comprise any thermoplastic polymeric material including, but not limited to, polyolefins, such as high density polyethylene, linear low density polyethylene, low density polyethylene, polypropylene; copolymers of olefins and vinyl monomers, such as copolymers of ethylene and vinyl acetate or vinyl chloride; polyamides; polyesters; polyvinyl alcohol and copolymers of olefins and acrylate monomers such as copolymers of ethylene and ethyl acrylate and ethylenemethacrylate. Films comprising mixtures of two or more of such polymeric materials may also be used. The machine direction (MD) and cross direction (CD) elongation of the starting film to be apertured should be at least 100% as determined according to ASTM Test No. D-882 as performed on an Instron test apparatus with a jaw speed of 50 inches/minute (127 cm/minute). The thickness of the starting film is preferably uniform and may range from about 0.5 to about 5 mils or about 0.0005 inch (0.0013 cm) to about 0.005 inch (0.076 cm). Coextruded films can be used, as can films that have been modified, e.g., by treatment with a surface active agent. The starting film can be made by any known technique, such as casting, extrusion, or blowing.

A method of aperturing the film involves placing the film onto the surface of a patterned support member. The film is subjected to a high fluid pressure differential as it is on the support member. The pressure differential of the fluid, which may be liquid or gaseous, causes the film to assume the surface pattern of the patterned support member. If the patterned support member has apertures therein, portions of the film overlying the apertures may be ruptured by the fluid pressure differential to create an apertured film. A method of forming an apertured film is described in detail in commonly owned US 5,827,597 to James et al.

Such a three dimensional apertured film is preferably formed by placing a thermoplastic film across the surface of an apertured support member with a pattern of macrofeatures and apertures. A stream of hot air is directed against the film to raise its temperature to cause it to be softened. A vacuum is then applied to the film to cause it to conform to the shape of the surface of the support member. Portions of the film lying over the apertures in the support member are ruptured to create apertures in the film.

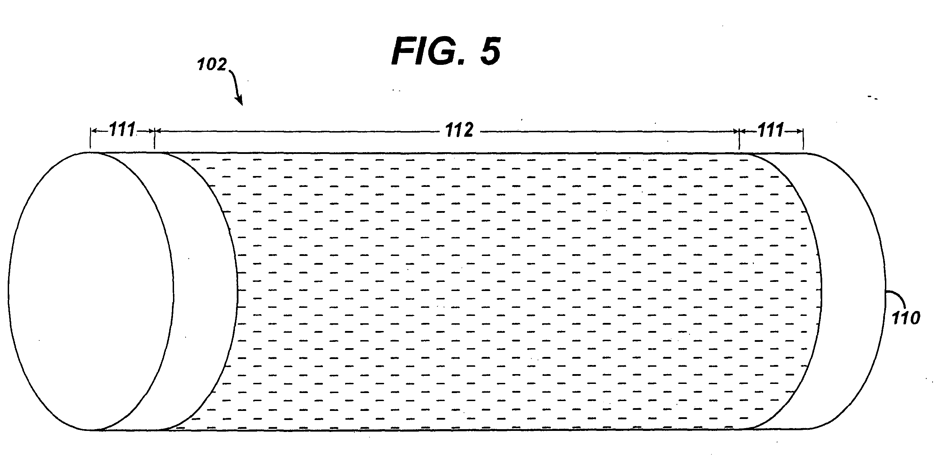

A suitable apertured support member for making these three-dimensional apertured films is a three-dimensional topographical support member made by laser sculpting a workpiece. A schematic illustration of an exemplary workpiece that has been laser sculpted into a three dimensional topographical support member is shown in Figure 5.

The workpiece 102 comprises a thin tubular cylinder 110. The workpiece 102 has non-processed surface areas 111 and a laser sculpted center portion 112. A preferred workpiece for producing the support member of this invention is a thin-walled seamless tube of acetal, which has been relieved of all residual internal stresses. The workpiece has a wall thickness of from 1-8 mm, more preferably from 2.5-6.5 mm. Exemplary workpieces for use in forming support members are one to six feet in diameter and have a length ranging from two to sixteen feet. However, these sizes are a matter of design choice. Other shapes and material compositions may be used for the workpiece, such as acrylics, urethanes, polyesters, high molecular weight polyethylene and other polymers that can be processed by a laser beam.

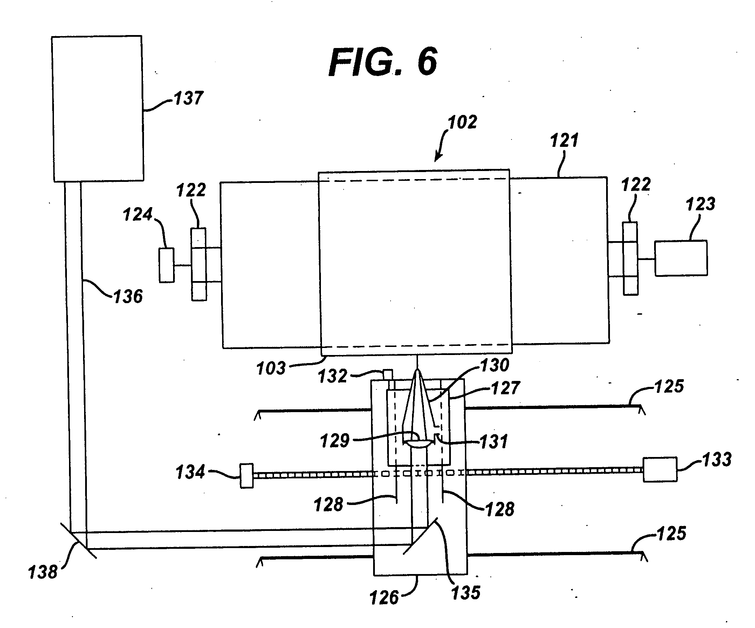

Referring now to Fig. 6, a schematic illustration of an apparatus for laser sculpting the support member is shown. A starting blank tubular workpiece 102 is mounted on an appropriate arbor, or mandrel 121 that fixes it in a cylindrical shape and allows rotation about its longitudinal axis in bearings 122. A rotational drive 123 is provided to rotate mandrel 121 at a controlled rate. Rotational pulse generator 124 is connected to and monitors rotation of mandrel 121 so that its precise radial position is known at all times.

Parallel to and mounted outside the swing of mandrel 121 is one or more guide ways 125 that allow carriage 126 to traverse the entire length of mandrel 121 while maintaining a constant clearance to the top surface 103 of workpiece 102. Carriage drive 133 moves the carriage along guide ways 125, while carriage pulse generator 134 notes the lateral position of the carriage with respect to workpiece 102. Mounted on the carriage is focusing stage 127. Focusing stage 127 is mounted in focus guide ways 128. Focusing stage 127 allows motion orthogonal to that of carriage 126 and provides a means of focusing lens 129 relative to top surface 103. Focus drive 132 is provided to position the focusing stage 127 and provide the focusing of lens 129.

Secured to focusing stage 127 is the lens 129, which is secured in nozzle 130. Nozzle 130 has means 131 for introducing a pressurized gas into nozzle 130 for cooling and maintaining cleanliness of lens 129. A preferred nozzle 130 for this purpose is described in US Patent 5,756,962 to James et al.

Also mounted on the carriage 126 is final bending mirror 135, which directs the laser beam 136 to the focusing lens 129. Remotely located is the laser 137, with optional beam bending mirror 138 to direct the beam to final beam bending mirror 135. While it would be possible to mount the laser 137 directly on carriage 126 and eliminate the beam bending mirrors, space limitations and utility connections to the laser make remote mounting far preferable.

When the laser 137 is powered, the beam 136 emitted is reflected by first beam bending mirror 138, then by final beam bending mirror 135, which directs it to lens 129. The path of laser beam 136 is configured such that, if lens 129 were removed, the beam would pass through the longitudinal center line of mandrel 121. With lens 129 in position, the beam may be focused above, below, at, or near top surface 103.

While this apparatus could be used with a variety of lasers, the preferred laser is a fast flow CO₂ laser, capable of producing a beam rated at up to 2500 watts. However, slow flow CO₂ lasers rated at 50 watts could also be used.

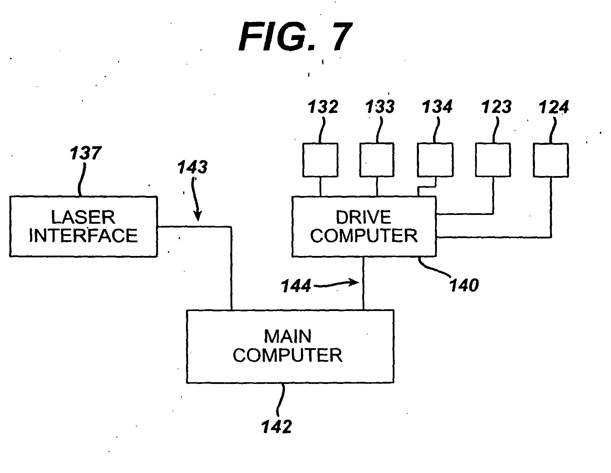

Figure 7 is a schematic illustration of the control system of the laser sculpting apparatus of Figure 6. During operation of the laser sculpting apparatus, control variables for focal position, rotational speed, and traverse speed are sent from a main computer 142 through connection 144 to a drive computer 140. The drive computer 140 controls focus position through focusing stage drive 132. Drive computer 140 controls the rotational speed of the workpiece 102 through rotational drive 123 and rotational pulse generator 124. Drive computer 140 controls the traverse speed of the carriage 126 through carriage drive 133 and carriage pulse generator 134. Drive computer 140 also reports drive status and possible errors to the main computer 142. This system provides positive position control and in effect divides the surface of the workpiece 102 into small areas called pixels, where each pixel consists of a fixed number of pulses of the rotational drive and a fixed number of pulses of the traverse drive. The main computer 142 also controls laser 137 through connection 143.

A laser sculpted three dimensional topographical support member may be made by several methods. One method of producing such a support member is by a combination of laser drilling and laser milling of the surface of a workpiece.

Methods of laser drilling a workpiece include percussion drilling, fire-on-the-fly drilling, and raster scan drilling.

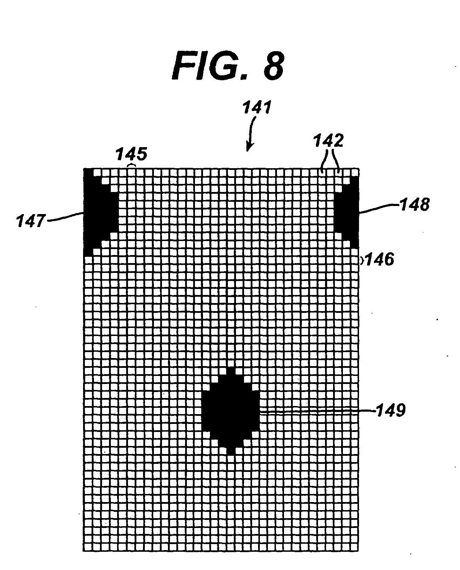

A preferred method is raster scan drilling. In this approach, the pattern is reduced to a rectangular repeat element 141 as depicted in FIG. 8. This repeat element contains all of the information required to produce the desired pattern. When used like a tile and placed both end-to-end and side-by-side, the larger desired pattern is the result.

This repeat element is further divided into a grid of smaller rectangular units or "pixels" 142. Though typically square, for some purposes, it may be more convenient to employ pixels of unequal proportions. The pixels themselves are dimensionless and the actual dimensions of the image are set during processing, that is, the width 145 of a pixel and the length 146 of a pixel are only set during the actual drilling operation. During drilling, the length of a pixel is set to a dimension that corresponds to a selected number of pulses from the carriage pulse generator 134. Similarly, the width of a pixel is set to a dimension that corresponds to the number of pulses from the rotational pulse generator 124. Thus, for ease of explanation, the pixels are shown to be square in Figure 8; however, it is not required that pixels be square, but only that they be rectangular.

Each column of pixels represents one pass of the workpiece past the focal position of the laser. This column is repeated as many times as is required to reach completely around workpiece 102. Each white pixel represents an off instruction to the laser, that is the laser is emitting no power, and each black pixel represents an on instruction to the laser, that is the laser is emitting a beam. This results in a simple binary file of 1's and 0's where a 1, or white, is an instruction for the laser to shut off and a 0, or black, is an instruction for the laser to turn on. Thus, in Figure 8, areas 147, 148 and 149 correspond to instructions for the laser to emit full power and will result in holes in the workpiece 102.

Referring back to Figure 7, the contents of an engraving file are sent in a binary form, where 1 is off and 0 is on, by the main computer 142 to the laser 137 via connection 143. By varying the time between each instruction, the duration of the instruction is adjusted to conform to the size of the pixel. After each column of the file is completed, that column is again processed, or repeated, until the entire circumference is completed. While the instructions of a column are being carried out, the traverse drive is moved slightly. The speed of traverse is set so that upon completion of a circumferential engraving, the traverse drive has moved the focusing lens the width of a column of pixels and the next column of pixels is processed. This continues until the end of the file is reached and the file is again repeated in the axial dimension until the total desired width is reached.

In this approach, each pass produces a number of narrow cuts in the material, rather than a large hole. Because these cuts are precisely registered to line up side-by-side and overlap somewhat, the cumulative effect is a hole.

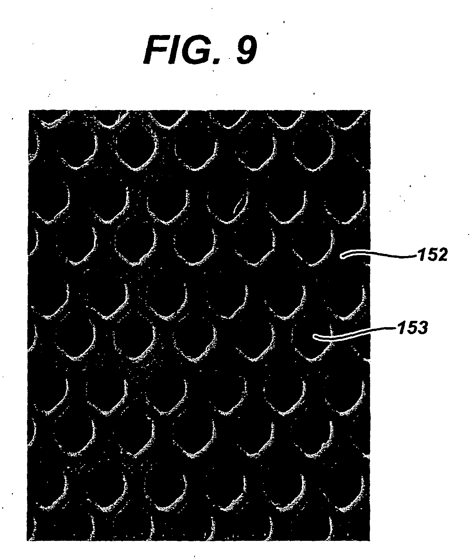

Figure 9 is a photomicrograph of a portion of a support member that has initially been raster scan drilled utilizing the file of Figure 8. The surface of the support member is a smooth planar surface 152 with a series of nested hexagonal holes 153.

A highly preferred method for making the laser sculpted three dimensional topographical support members is through laser modulation. Laser modulation is carried out by gradually varying the laser power on a pixel by pixel basis. In laser modulation, the simple on or off instructions of raster scan drilling are replaced by instructions that adjust on a gradual scale the laser power for each individual pixel of the laser modulation file. In this manner a three dimensional structure can be imparted to the workpiece in a single pass over the workpiece.

Laser modulation has several advantages over other methods of producing a three dimensional topographical support member. Laser modulation produces a one-piece, seamless, support member without the pattern mismatches caused by the presence of a seam. With laser modulation, the support member is completed in a single operation instead of multiple operations, thus increasing efficiency and decreasing cost. Laser modulation eliminates problems with the registration of patterns, which can be a problem in a multi-step sequential operation. Laser modulation also allows for the creation of topographical features with complex geometries over a substantial distance. By varying the instructions to the laser, the depth and shape of a feature can be precisely controlled and features that continuously vary in cross section can be formed. The regular positions of the apertures and macrofeatures relative to one another can be maintained.

Referring again to Figure 7, during laser modulation the main computer 142 may send instructions to the laser 137 in other than a simple "on" or "off" format. For example, the simple binary file may be replaced with an 8 bit (byte) format, which allows for a variation in power emitted by the laser of 256 possible levels. Utilizing a byte format, the instruction "11111111" instructs the laser to turn off, "00000000" instructs the laser to emit full power, and an instruction such as "10000000" instructs the laser to emit one-half of the total available laser power.

A laser modulation file can be created in many ways. One such method is to construct the file graphically using a gray scale of a 256 color level computer image. In such a gray scale image, black can represent full power and white can represent no power with the varying levels of gray in between representing intermediate power levels. A number of computer graphics programs can be used to visualize or create such a laser-sculpting file. Utilizing such a file, the power emitted by the laser is modulated on a pixel by pixel basis and can therefore directly sculpt a three dimensional topographical support member. While an 8-bit byte format is described here, other levels, such as 4 bit, 16 bit, 24 bit or other formats can be substituted.

A suitable laser for use in a laser modulation system for laser sculpting is a fast flow CO₂ laser with a power output of 2500 watts, although a laser of lower power output could be used. Of primary concern is that the laser must be able to switch power levels as quickly as possible. A preferred switching rate is at least 10 kHz and even more preferred is a rate of 20 kHz. The high power-switching rate is needed to be able to process as many pixels per second as possible.

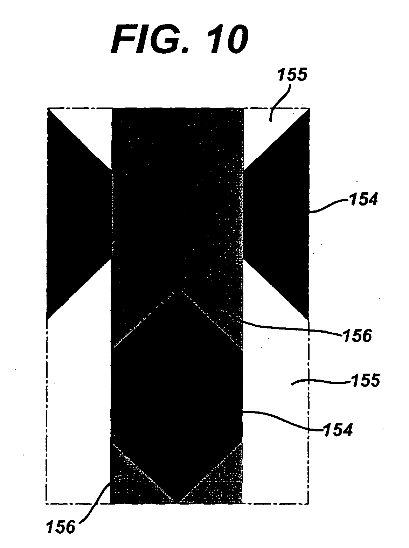

Figure 10 shows a graphical representation of a laser modulation file to produce a support member using laser modulation. The support member made with the file of Figure 10 is used to make the three-dimensional apertured film shown in Figure 2. In Figure 10, the black areas 154 indicate pixels where the laser is instructed to emit full power, thereby creating a hole in the support member, which corresponds to apertures 12 in the three-dimensional apertured film 20 illustrated in Figure 2. Likewise, white areas 155 in Figure 10 indicate pixels where the laser receives instructions to turn off, thereby leaving the surface of the support member intact. These intact areas of the support member correspond to the macrofeatures 14 of the three-dimensional apertured film 20 of Figure 2. The gray area 156 in Figure 10 indicates pixels where the laser is instructed to emit partial power and produce a lower region on the support member. This lower region on the support member corresponds to lower region 16 on the three-dimensional apertured film 20 of Figure 2.

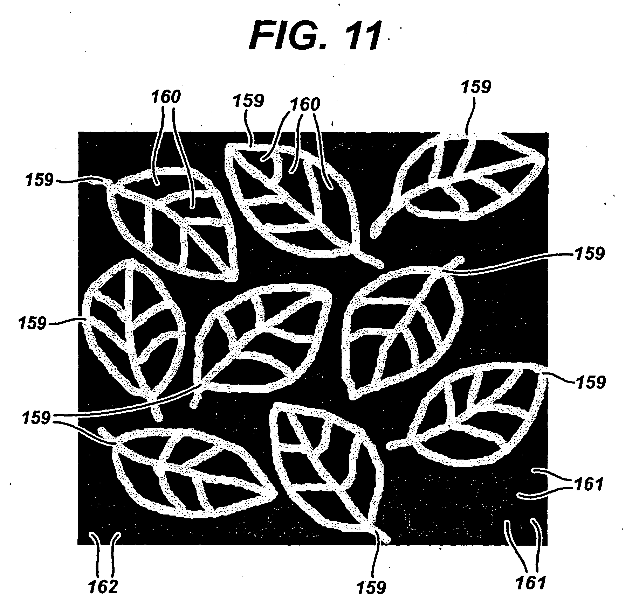

Figure 11 shows a graphical representation of a laser modulation file to produce a support member using laser modulation. As in the laser-drilling file of Figure 8, each pixel represents a position on the surface of the workpiece. Each row of pixels represents a position in the axial direction of the workpiece to be sculpted. Each column of pixels represents a position in the circumferential position of the workpiece. Unlike the file of Figure 8 however, each of the laser instructions represented by the pixels is no longer a binary instruction, but has been replaced by 8 bit or gray scale instructions. That is, each pixel has an 8-bit value, which translates to a specific power level.

Figure 11 is a graphical representation of a laser modulation file to produce a support member using laser modulation. The file shows a series of nine leaf-like structures 159, which are shown in white. The leaves are a series of white pixels and are instructions for the laser to be off and emit no power. Leaves of these shapes, therefore, would form the uppermost surface of the support member after the pattern has been sculpted into it. Each leaf structure contains a series of six holes 160, which are defined by the stem-like structures of the leaves and extend through the thickness of the workpiece. The holes 160 consist of an area of black pixels, which are instructions for the laser to emit full power and thus drill through the workpiece. The leaves are discrete macrofeatures, i.e., by themselves they do not form a flat planar structure, as no leaf interconnects with any other leaf. The background pattern of this structure consists of a close-packed staggered pattern of hexagonal black areas 161, which are also instructs for the laser to emit full power and drill a hole through the workpiece. The field 162, which defines holes 161, is at a laser power level that is neither fully on nor fully off. This produces a second planar area, which is below the uppermost surface of the workpiece as defined by the off instructions of the white areas of the leaves.

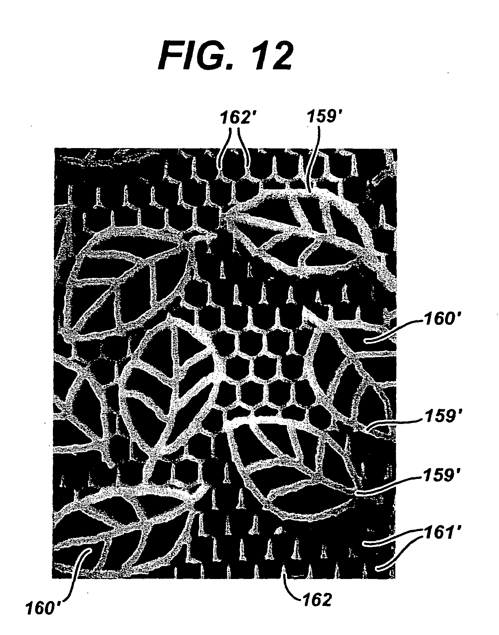

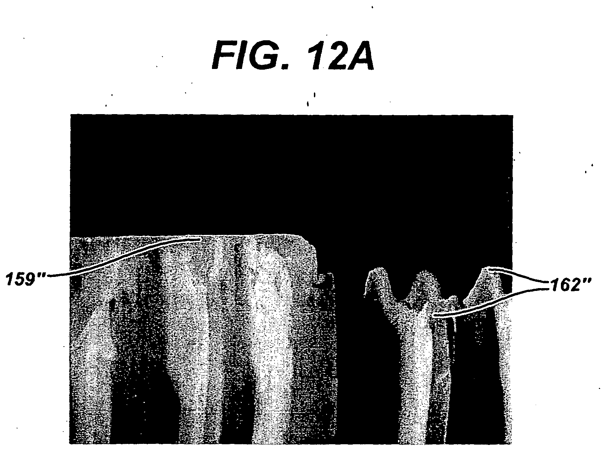

Figure 12 is a photomicrograph of a laser sculpted three dimensional topographical support member produced by laser modulation utilizing the laser modulation file depicted in Figure 11. Figure 12A is a cross-sectional view of the support member of Figure 12. Regions 159' of Figure 12 and 159" of Figure 12A correspond to the leaf 159 of Figure 11. The white pixel instructions of areas 159 of Figure 11 have resulted in the laser emitting no power during the processing of those pixels. The top surface of the leaves 159' and 159" correspond to the original surface of the workpiece. Holes 160' in Figure 12 correspond to the black pixel areas 160 of Figure 11, and in processing these pixels the laser emits full power, thus cutting holes completely through the workpiece. The background film 162' of Figure 12 and 162" of Figure 12A correspond to the pixel area 162 of Figure 11. Region 162' results from processing the pixels of Figure 11 with the laser emitting partial power. This produces an area in the support member that is lower than the original surface of the workpiece and that is thus lower than the top surface of the leaves. Accordingly, the individual leaves are discrete macrofeatures, unconnected to each other.

Figures 13 and 13A are photomicrographs of a three-dimensional apertured film that has been produced on the support member of Figures 12 and 12A. The apertured film has raised apertured leaf-shaped macrofeatures 176 and 176', which correspond to the leaves 159' and 159" of the support member of Figures 12 and 12A. Each of the leaves is discrete and disconnected from all the other leaves. Each leaf contains apertures, i.e., each leaf is an apertured macrofeature. The plane defined by the uppermost surfaces of all the leaf shaped regions 176 and 176' is the uppermost surface of a plurality of disconnected macrofeatures. The background apertured regions 177 and 177' define a region that is at a lower depth in the film than the leaf shaped regions. This gives the visual impression that the leaves are embossed into the film.

The laser sculpted support members of Figures 9, 12, and 12A have simple geometries. That is, successive cross-sections, taken parallel to the uppermost surface of the support member, are essentially the same for a significant depth through the thickness of the support member. For example, referring to Figure 9, successive cross-sections of this support member taken parallel to the surface of the support member are essentially the same for the thickness of the support member. Similarly, cross-sections of the support member of Figures 12 and 12A are essentially the same for the depth of the leaves and are essentially the same from the base of the leaves through the thickness of the support member.

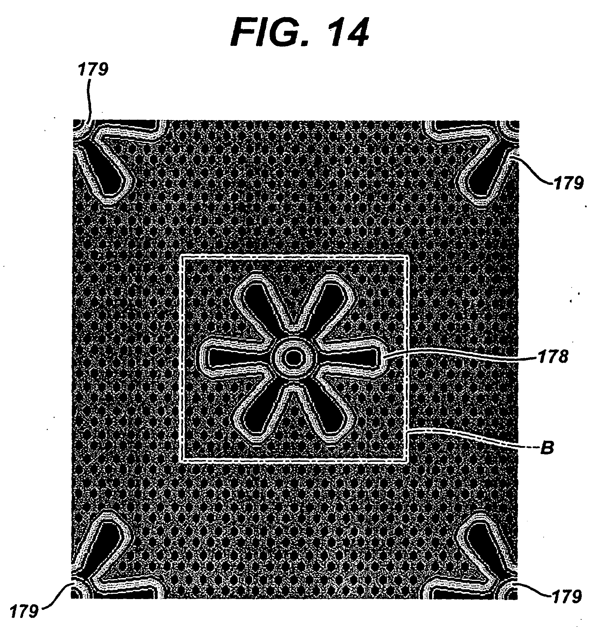

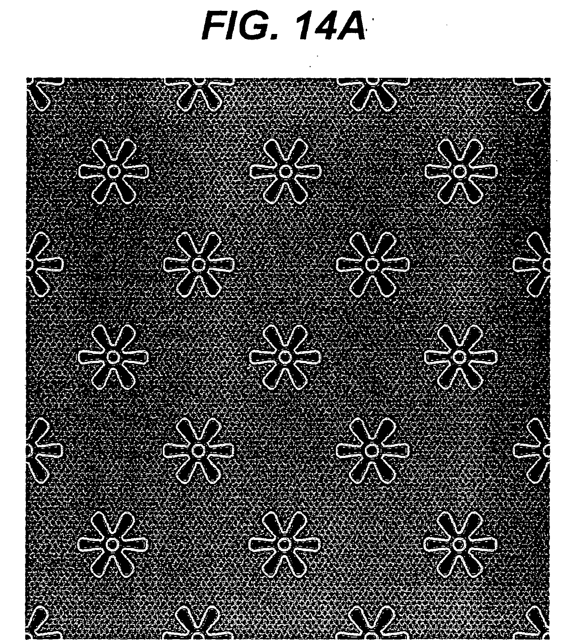

Figure 14 is a graphical representation of another laser modulation file to produce a laser sculpted support member using laser modulation. The file contains a central floral element 178 and four elements 179, each of which constitutes a quarter of a floral element 178, which combine when the file is repeated during laser sculpting. Figure 14A is a 3 repeat by 3 repeat graphical representation of the resulting pattern when the file of Figure 14 is repeated.

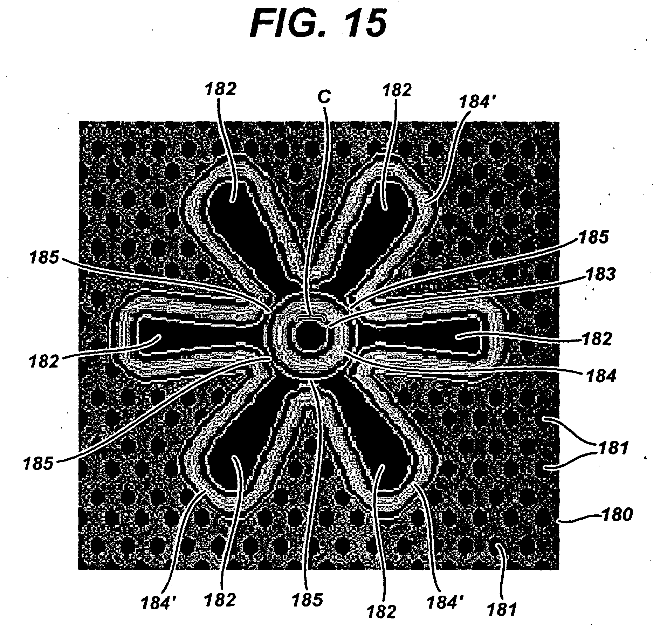

Figure 15 is a magnified view of the area B of Figure 14. The gray area represents a region of pixels instructing the laser to emit partial power. This produces a planar area below the surface of the workpiece. Contained in gray region 180 is a series of black areas 181 which are pixels instructing the laser to emit full power and drill a series of hexagonal shaped holes through the thickness of the workpiece. Central to Figure 15 is the floral element corresponding to the floral element 178 of Figure 14. The floral element consists of a center region 183 and six petal shaped regions 182 which again represent instructions for the laser to emit full power and drill a hole through the thickness of the workpiece. Defining the outside edge of the center region 183 is region 184. Defining the outside edge of the petal regions 182 is region 184'. Regions 184 and 184' represent a series of instructions for the laser to modulate the emitted power. The central black region 183 and its outside edge region 184 are joined to the region 184' by region 185 which represents instructions for the laser to emit the same power level as the background area 180.

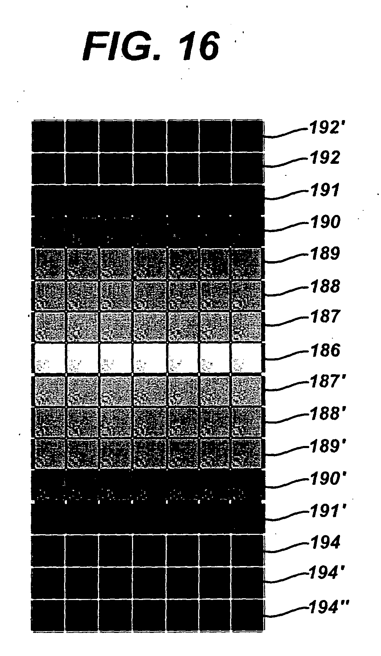

Figure 16 is an enlarged graphical representation of portion C of region 184 of Figure 15 which forms the outline of the center region 183 of Figure 15. The portion C contains a single row of white pixels 186 which instruct the laser to turn off. This defines part of the uppermost surface of the support member that remains after processing. The rows of pixels 187 and 187' instruct the laser to emit partial power. The rows 188, 189, 190, and 191 and the rows 188', 189' 190', and 191' instruct the laser to emit progressively increased levels of power. Rows 192 and 192' instruct the laser to emit the power level also represented by region 185 of Figure 15. Rows 194, 194', and 194" instruct the laser to emit full power and form part of region 183 of Figure 15.

As each column of Figure 16 is processed the laser emits the partial power represented by rows 192 and 192'. Rows 191, 190, 189, 188, and 187 instruct the laser to progressively decrease the power emitted, until row 186 is processed and the laser is instructed to not emit power. The rows 187', 188', 189', 190', and 191' then instruct the laser to again progressively increase the power emitted. Rows 194, 194', and 194" instruct the laser to again emit full power to begin drilling through the workpiece. This results in the creation of a disconnected macrofeature, which slopes from the background plane to the surface of the workpiece and then slopes back to the hole area, thus producing a radiused shape.

Depending on the size of the pixels as defined during processing, and the variation in emitted laser power for each row, the size and shape of the resulting laser sculpted feature can be changed. For example, if the variation in power level for each row of pixels is small, then a relatively shallow rounded shape is produced; conversely, if the variation in power level for each row of pixels is greater, then a deep, steep shape with a more triangular cross-section is produced. Changes in pixel size also affect the geometry of the features produced. If the pixel size is kept smaller than the actual diameter of the focused laser beam emitted, then smooth blended shapes will be produced.

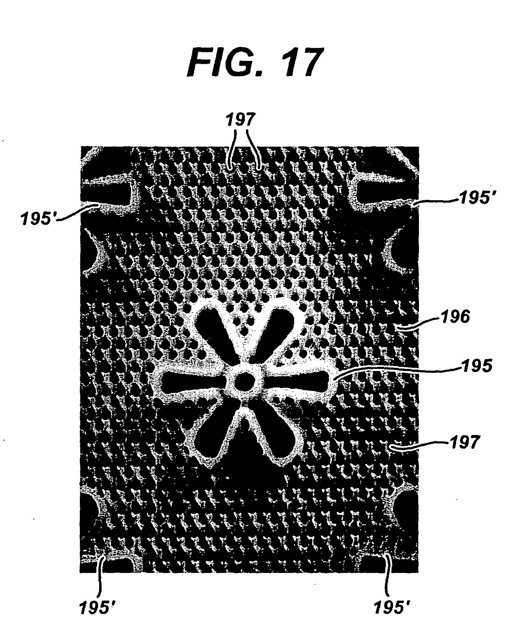

Figure 17 is a photomicrograph of the laser sculpted support member resulting from the processing of the file of Figure 14 by laser modulation. The photomicrograph shows a raised floral element 195, which corresponds to the floral element 178 of Figure 14 and the floral element of Figure 15. The photomicrograph also shows portions of additional floral elements 195'. Raised floral element 195 originates in the planar region 196, which contains holes 197. Floral elements 195 and 195' are disconnected from one another and thus do not form a continuous planar region.

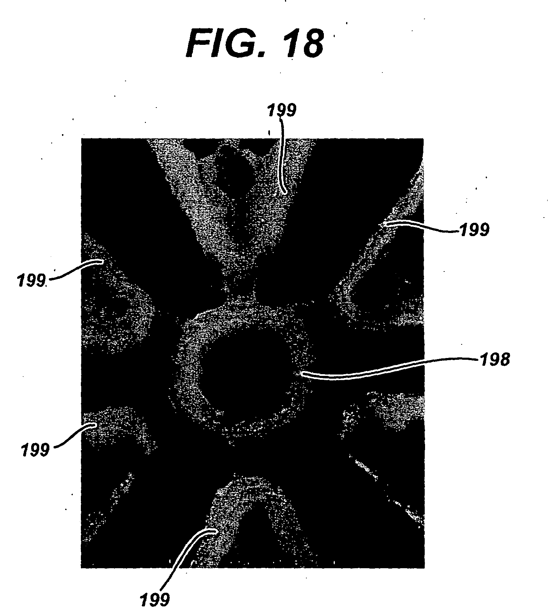

Figure 18 is an enlarged photomicrograph of a portion of the floral element 195 of Figure 17. The center circular element 198 is the area produced by the laser modulation instructions contained in region 184 of Figure 15. The elements 199 are parts of the petal elements of the floral element 195 of Figure 17. These petal elements are produced by pixel instructions depicted in region 184' of Figure 15. These elements demonstrate an example of a type of complex geometry that can be created by laser modulation. The central circular element has a semicircular cross section. That is, any one of a series of cross-sectional planes taken parallel to the original surface of the workpiece, i.e., through the depth will differ from any other of such cross-sectional planes.

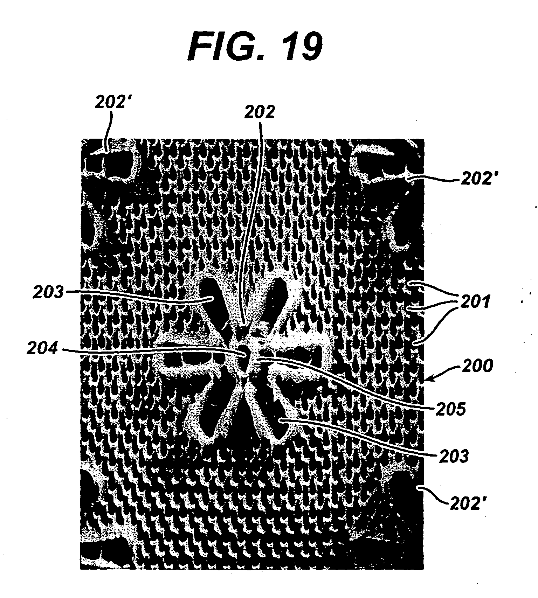

Figure 19 is a photomicrograph of the upper surface of a film produced on the support member of Figure 17. The film has an apertured planar area 200, containing holes 201 that corresponds to planar region 196 of Figure 17. Extending above the planar area are floral areas 202 and 202', which correspond to floral elements 195 and 195', respectively, of Figure 17. The floral areas 202 and 202' give the resulting apertured film an embossed appearance in a single operation. In addition, the floral areas define additional larger holes 203 and 204 to improve fluid transmission properties.

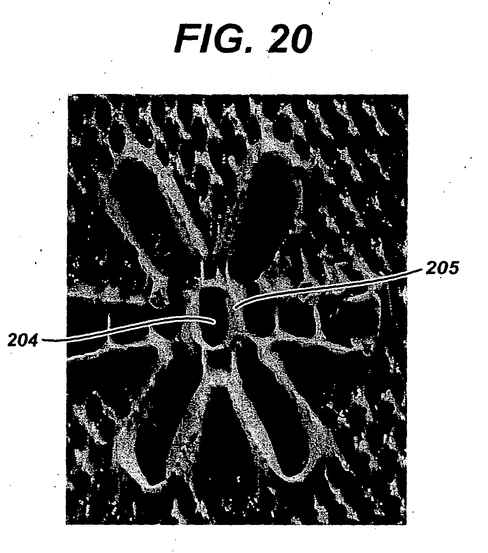

Figure 20 is an enlargement of the floral area 202 of Figure 19. The floral area comprises hole 204 and the surrounding circular element 205. Element 205 of Figures 19 and 20 has a complex geometry in that it has a semicircular cross-section. Again, successive cross-sections taken parallel to the surface of the film taken through its depth are different.

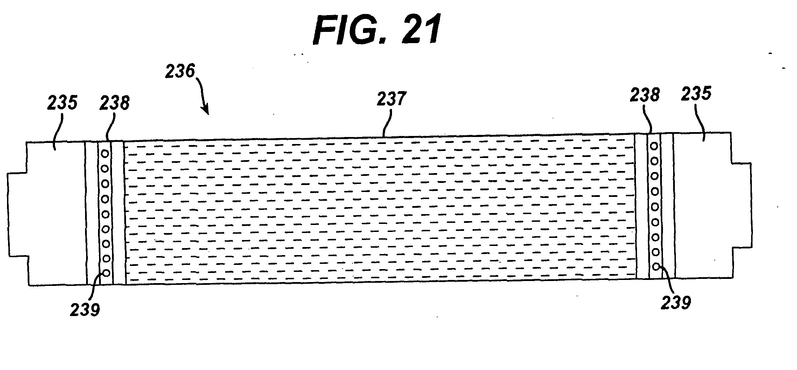

Upon completion of the laser sculpting of the workpiece, it can be assembled into the structure shown in Figure 21 for use as a support member. Two end bells 235 are fitted to the interior of the workpiece 236 with laser sculpted area 237. These end bells can be shrink-fit, press-fit, attached by mechanical means such as straps 238 and screws 239 as shown; or by other mechanical means. The end bells provide a method to keep the workpiece circular, to drive the finished assembly, and to fix the completed structure in the aperturing apparatus.

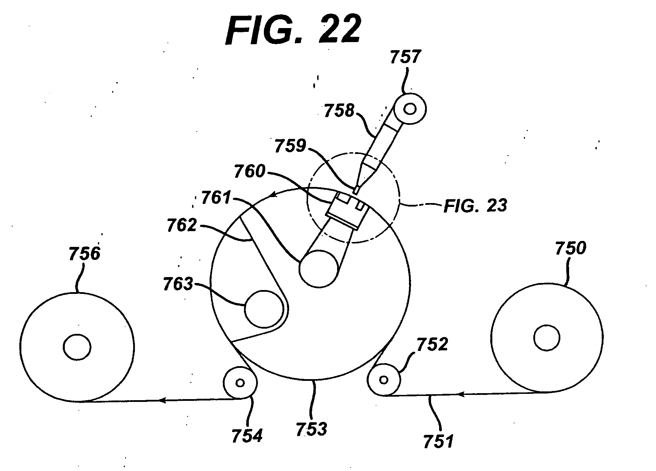

A preferred apparatus for producing such three dimensional apertured films is schematically depicted in Figure 22. As shown here, the support member is a rotatable drum 753. In this particular apparatus, the drum rotates in a counterclockwise direction. Positioned outside drum 753 is a hot air nozzle 759 positioned to provide a curtain of hot air to impinge directly on the film supported by the laser sculpted support member. Means is provided to retract hot air nozzle 759 to avoid excessive heating of the film when it is stopped or moving at slow speed. Blower 757 and heater 758 cooperate to supply hot air to nozzle 759. Positioned inside the drum 753, directly opposite the nozzle 759, is vacuum head 760. Vacuum head 760 is radially adjustable and positioned so as to contact the interior surface of drum 753. A vacuum source 761 is provided to continuously exhaust vacuum head 760.

Cooling zone 762 is provided in the interior of and contacting the inner surface of drum 753. Cooling zone 762 is provided with cooling vacuum source 763. In cooling zone 762, cooling vacuum source 763 draws ambient air through the apertures made in the film to set the pattern created in the aperturing zone. Vacuum source 763 also provide means of holding the film in place in cooling zone 762 in drum 753, and provides means to isolate the film from the effects of tension produced by winding up the film after its aperturing.

Placed on top of laser sculpted support member 753 is a thin, continuous, uninterrupted film 751 of thermoplastic polymeric material.

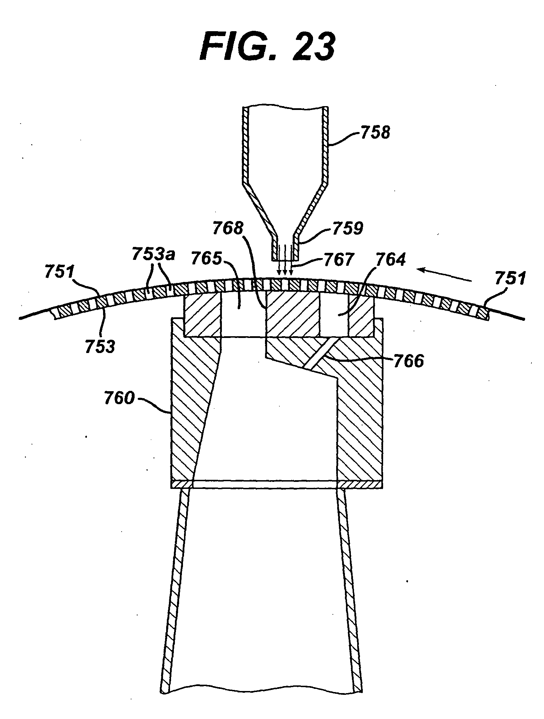

An enlargement of the circled area of Figure 22 is shown in Figure 23. As shown in this embodiment, vacuum head 760 has two vacuum slots 764 and 765 extending across the width of the film. However, for some purposes, it may be preferred to use separate vacuum sources for each vacuum slot. As shown in Figure 23, vacuum slot 764 provides a hold down zone for the starting film as it approaches air knife 758. Vacuum slot 764 is connected to a source of vacuum by a passageway 766. This anchors the incoming film 751 securely to drum 753 and provides isolation from the effects of tension in the incoming film induced by the unwinding of the film. It also flattens film 751 on the outer surface of drum 753. The second vacuum slot 765 defines the vacuum aperturing zone. Immediately between slots 764 and 765 is intermediate support bar 768. Vacuum head 760 is positioned such that the impingement point of hot air curtain 767 is directly above intermediate support bar 768. The hot air is provided at a sufficient temperature, a sufficient angle of incidence to the film, and at a sufficient distance from the film to cause the film to become softened and deformable by a force applied thereto. The geometry of the apparatus ensures that the film 751, when softened by hot air curtain 767, is isolated from tension effects by hold-down slot 764 and cooling zone 762 (Figure 22). Vacuum aperturing zone 765 is immediately adjacent hot air curtain 767, which minimizes the time that the film is hot and prevents excessive heat transfer to support member 753.

Referring to Figures 22 and 23, a thin flexible film 751 is fed from a supply roll 750 over idler roll 752. Roll 752 may be attached to a load cell or other mechanism to control the feed tension of the incoming film 751. The film 751 is then placed in intimate contact with the support member 753. The film and support member then pass to vacuum zone 764. In vacuum zone 764 the differential pressure further forces the film into intimate contact with support member 753. The vacuum pressure then isolates the film from the supply tension. The film and support member combination then passes under hot air curtain 767. The hot air curtain heats the film and support member combination, thus softening the film.

The heat-softened film and the support member combination then pass into vacuum zone 765 where the heated film is deformed by the differential pressure and assumes the topography of the support member. The heated film areas that are located over open areas in the support member are further deformed into the open areas of the support member. If the heat and deformation force are sufficient, the film over the open areas of the support member is ruptured to create apertures.

The still-hot apertured film and support member combination then passes to cooling zone 762. In the cooling zone a sufficient quantity of ambient air is pulled through the now-apertured film to cool both the film and the support member.

The cooled film is then removed from the support member around idler roll 754. Idler roll 754 may be attached to a load cell or other mechanism to control winding tension. The apertured film then passes to finish roll 756, where it is wound up.

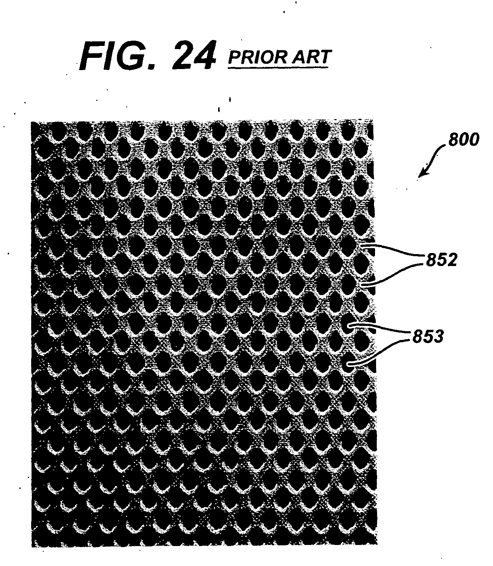

Figure 24 is a photomicrograph of an apertured film 800 of the prior art that was produced on a support member that has been raster scan drilled utilizing the file of Figure 9. The surface of this apertured film is a planar surface 852 with a series of nested hexagonal holes 853.

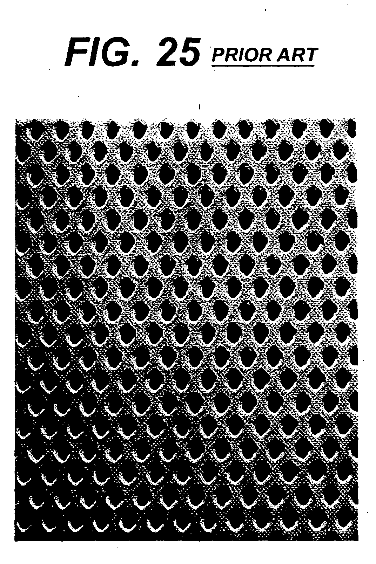

Figure 25 is a photomicrograph of another apertured film of the prior art that was produced on another support member that was produced by raster scan drilling. The surface of this apertured film is also characterized by a planar surface and a series of nested hexagonal holes that are larger than those shown in Figure 24.

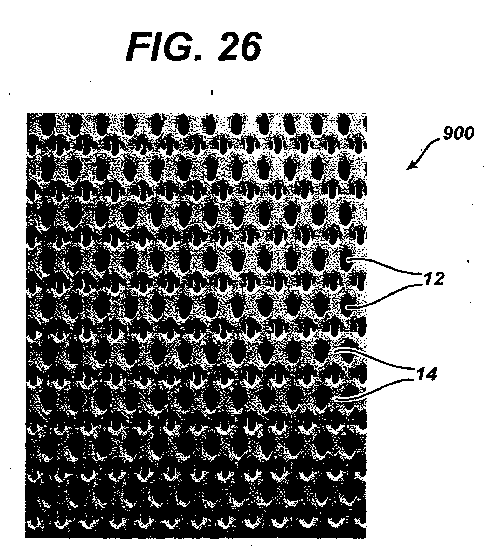

Figure 26 is a photomicrograph of a further embodiment of a three-dimensional apertured film of the present invention with an arrangement of apertures and macrofeatures. The film 880 of Figure 26 has apertures 12 arranged with macrofeatures 14. All of the apertures 12 and macrofeatures 14 are arranged together so that their relative positions to one another are regular.

While the method of forming a three dimensional apertured film has been described using a hot air curtain as the mechanism to heat the film, any suitable method such as infrared heating, heated rolls, or the like may be employed to produce an apertured film using the laser sculpted three-dimensional topographical support member of this invention.

In another method for producing an apertured film the incoming film supply system can be replaced with a suitable extrusion system. In this case the extrusion system provides a film extrudate; which, depending on the extrudate temperature, can either be cooled to a suitable temperature by various means such as cold air blast or chilled roll prior to contacting the three dimensional topographical support or be brought in direct contact with the three dimensional topographical support. The film extrudate and forming surface are then subjected to the same vacuum forming forces as described above without the need to heat the film to soften the film to make it deformable.

Figure 27 is a cross-section of a two layer structure according to the invention. The structure comprises a body contacting layer 500, in this case a nonwoven, overlying a second layer 501, also a nonwoven. Second layer 501 comprises a plurality of macrofeatures 14 projecting in the direction of the body contacting layer 500. The second layer 501 may be secured to the body contacting layer 500 using a suitable adhesive known to those skilled and then passing the two layer structure through a nip roll or the like.

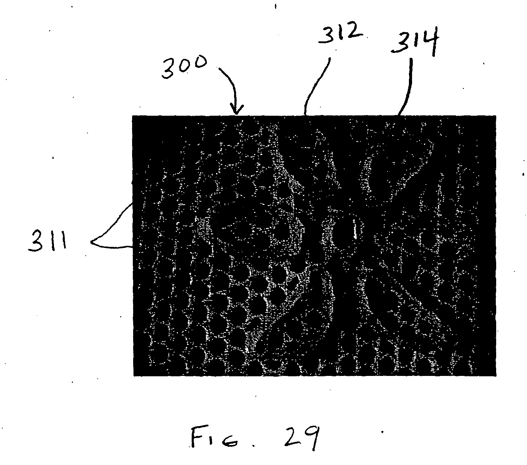

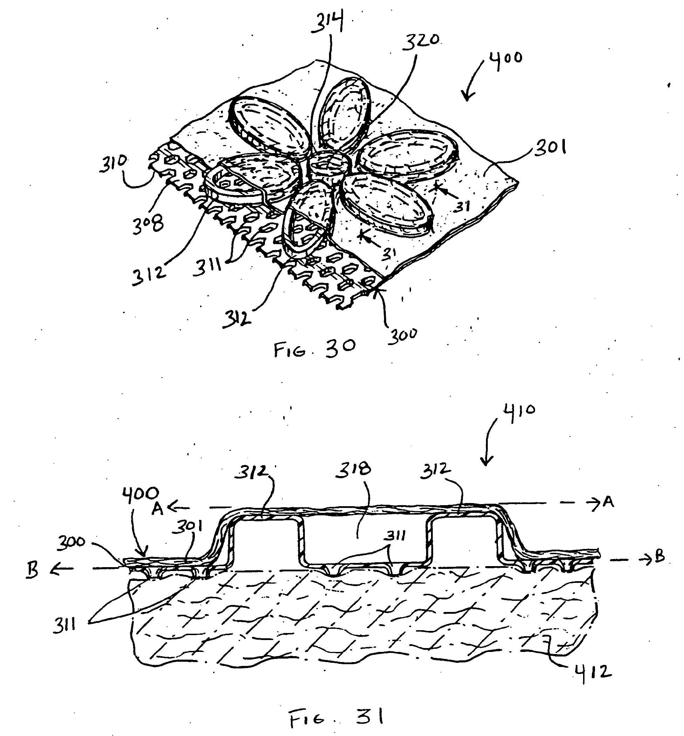

Figure 29 is a photomicrograph of a portion of an apertured film 300 produced in accordance with the invention. Figure 30 is a partially cut away perspective view of a two layer structure 400 according to the invention. As shown, the structure 400 includes a body contacting layer 301, in this case a nonwoven, overlying a second layer 300, in this case the apertured film 300 shown in Figure 29.

The second layer 300 comprises a substantially planar area 303 having a first surface 308, opposed second surface 310 and a plurality of apertures 311 extending from the first surface 308 to the second surface 310.

The second layer 300 further includes a plurality of substantially oval macrofeatures 312 projecting from the first surface 308 of the planar area 303 in the direction of the body contacting layer 302. The second layer also includes a central circular macrofeature 314. The substantially oval macrofeatures 312 are arranged in an array around the circular macrofeature 314 such that the macrofeatures 312 and macrofeature 314 collectively give the visual appearance of a flower. In this manner, macrofeatures 312 and 314 cooperate to define a visual design element.

Although only a single "flower" is depicted in Figure 29, it is understood that Fig. 29 only shows a portion of the apertured film and the complete apertured film would preferably include a plurality of such "flower" designs by having plurality of the oval macrofeatures 312 and a plurality of the circular macrofeatures 314 to give the appearance of a plurality of such "flowers". Likewise, Figure 30 only depicts a portion of a two layer structure according to the invention and the complete two layer structure would preferably include a plurality of such flower designs. Moreover, although a flower design has been depicted as the visual design element it will be apparent that numerous other design elements could be created using macrofeatures of the type disclosed herein.

Fig. 31 is a cross-section view of an absorbent article 410 including the two layer structure 400 shown in Fig. 29, taken along line 31-31. The absorbent article 410 further includes an absorbent core 412 that is in fluid communication with the two layer structure 400.

As best seen in Fig. 31, in those areas of the two layer structure 400 that are located outside the macrofeatures 312 and 314, the second layer 300 is substantially in surface to surface contact with body contacting layer 301 as shown. Further, the second layer 300 contacts the body contacting layer 301 at each one of the plurality of macrofeatures 312 and 314.

In the areas defined within the macrofeatures 312 and 314, i.e. within the areas designated 318 and 320 respectively, the body contacting layer 301 is arranged in a first substantially planar plane A and the second layer 300 is arranged in a second substantially planar plane B that is spaced from the first plane A. In this manner, in those areas defined within macrofeatures 312 and 314, the body contacting layer 301 is arranged in spaced relationship relative to the second layer 300.

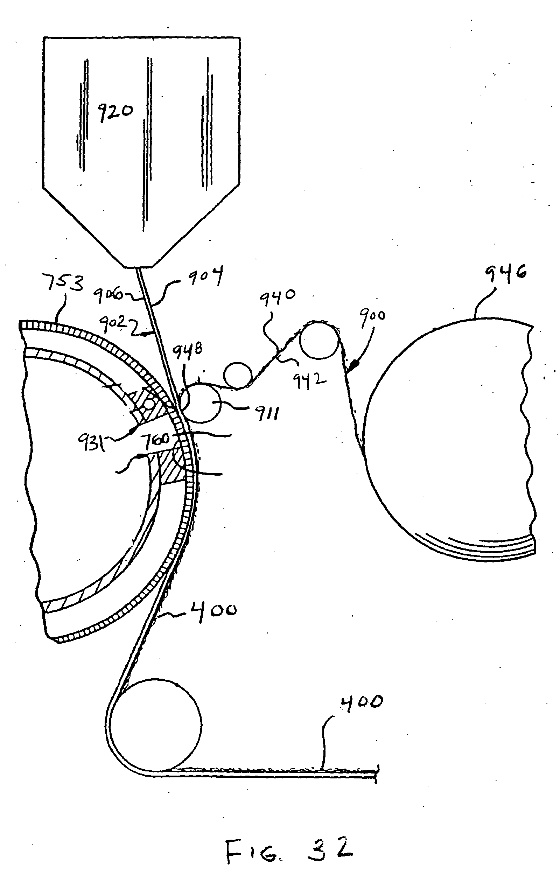

The two layer structure 400 shown in Figure 30 is preferably formed as vacuum formed laminate using the process as substantially shown and described in U.S. Patent 6,303,208. The specific technique used to form the two layer structure 400 will be described with reference to Figure 32. Figure 32 is a simplified schematic illustration showing a process to adhere a fibrous carrier material 900 (e.g. a nonwoven) onto a molten or semi-molten film material having a top surface 904 and a bottom surface 906. The fibrous material 900 is applied through a nip roll 911 to form the two layer laminate structure 600.

As shown, the film material 902 is dispensed from a film die 920 onto the support member 753. The film material 902 is delivered at an elevated temperature. The film material 902 is formed and perforated by passing the material over the support member 753 and applying a pressure differential by a vacuum head 760. As the film material 901 is extruded from the die 920 the film material comes into contact with the rotating surface of the support member 753. The rotating surface of the support member 753 moves continues portions of the film material across the vacuum head 760 such that the film is deformed to assume the topography of the support member 753.

The fibrous material 900 has first surface 940, which is brought into contact with the top surface of the film 904 of the film 902. The fibrous material 10 has a second surface 942 that is opposed to the first surface 904. A dispensing means 946 transfers the fibrous material 900 to an impingement or lamination point 948 where the fibrous material 900 and film material 902 contact each other to form the laminate 400. In the embodiment shown, the fibrous material 900 contacts the film 902 at the impingement point 948 prior to the leading edge 931 of the vacuum head 760. After passing under the impingement point 948 the film material 902 and fibrous material 900 pass over the vacuum chamber to thereby define the apertures and macrofeatures in the film material 902.

The two layer structures described above may advantageously be used as a cover/transfer layer of an absorbent article, such as a sanitary napkin, pantiliner, diaper, incontinence pad, or other similar product for absorbing exudates from the body, such as menses, urine, feces, or sweat. Preferably, the absorbent article is a sanitary napkin or a pantiliner. Such sanitary napkin or pantiliner may have an approximately rectangular, oval, dogbone, or peanut shape. Depending on the nature of the absorbent article, its size may vary. For example, sanitary napkins typically have a caliper of about 1.4 to about 5 mm, a length of about 8 to about 41 centimeters (cm), and a width of about 2.5 to about 13 cm. Pantiliners typically have a caliper of less than about 5 mm, a length of less than about 20 cm, and a width of less than about 8 cm.

The two layer structures described above are preferably placed over a suitable absorbent core, which is typically comprised of a loosely associated absorbent hydrophilic material such as cellulose fibers, including wood pulp, regenerated cellulose fibers or cotton fibers, or other absorbent materials generally known in the art, including acrylic fibers, polyvinyl alcohol fibers, peat moss and superabsorbent polymers.

The absorbent article may further comprise a backsheet that is substantially or completely impermeable to liquids, the exterior of which forms the garment-facing surface of the article. The backsheet may comprise any thin, flexible, body fluid impermeable material such as a polymeric film, for example, polyethylene, polypropylene, or cellophane. Alternatively, the backsheet may be a normally fluid permeable material that has been treated to be impermeable, such as impregnated fluid repellent paper or nonwoven fabric material, or a flexible foam, such as polyurethane or cross-linked polyethylene. The thickness of the backsheet when formed from a polymeric film typically is about 0.025 mm to 0.051 mm. A variety of materials are known in the art for use as backsheet, and any of these may be used. The backsheet may be breathable, i.e., a film that is a barrier to liquids but permits gases to transpire. Materials for this purpose include microporous films in which microporosity is created by stretching an oriented film. Single or multiple layers of permeable films, fabrics, and combinations thereof that provide a tortuous path, and/or whose surface characteristics provide a liquid surface repellent to the penetration of liquids may also be used to provide a breathable backsheet.

A cross-sectional view of an absorbent article comprising a two layer structure according to the invention is shown in Figure 28. The two layer structure is used as a cover/transfer layer. The absorbent article comprises a backsheet 503. Overlying the backsheet is an absorbent core 502. Overlying the absorbent core is the two layer structure 504. Two layer structure 504 comprises a nonwoven first or body contacting layer 500 over a second layer 501 that is an apertured film. The apertured film comprises a disconnected macrofeatures 14 and apertures 12.

The absorbent article may comprise other known materials, layers, and additives, such as adhesives, release paper, foam layers, net-like layers, perfumes, medicaments, moisturizers, and the like, many examples of which are known in the art.

### EXAMPLES

Structures of the present invention comprising a fluid permeable first layer in fluid communication with a fluid permeable second layer, wherein the layers contact one another substantially only through a plurality of disconnected macrofeatures have favorable fluid handling properties. In particular, disposable absorbent products with a component layer having a plurality of disconnected macrofeatures have a low Fluid Penetration time. Additionally, disposable absorbent products comprising apertured film having a plurality of disconnected macrofeatures exhibit a Repeat Insult Time that increases less than about 40% over six insults.

Structures according to the present invention comprising an apertured film having a plurality of disconnected macrofeatures (Examples 1, 2, and 3) and structures containing samples of conventional) apertured film (Prior Art 1 and 2 were compared as transfer layers using the Fluid Penetration Test and the Repeat Insult Test. The test fluid used for the Fluid Penetration Test and the Repeat Insult Test was a synthetic menstrual fluid having a viscosity of 30 centipoise at 1 radian per second.

Test assemblies were made from Examples 1-3 and Prior Art 1 and 2 using cover layer, absorbent core and barrier from the commercially available sanitary napkin, Stayfree Ultra Thin Long with Wings, distributed by Personal Products Company Division of McNeil-PPC, Inc. Skillman, NJ. The cover layer is a thermally bonded polypropylene fabric; the absorbent core is a material containing superabsorbent polymer and the barrier is a pigmented polyethylene film. The cover layer and transfer layers were each carefully peeled away from the product exposing the absorbent core which remained adhesively attached to the barrier film. Next, a piece of transfer layer material to be tested was cut to a size approximately 200 mm long by at least the width of the absorbent core and a pressure sensitive hot melt adhesive such as HL-1471xzp commercially available from HB Fuller Corporation, St. Paul, MN 55110, was applied to the side of the transfer layer material oriented adjacent to the exposed surface of the absorbent core. Adhesive was applied to the material to be tested by transfer from release paper which was coated with approximately 1.55 gram per square meter. The transfer layer material to be tested was oriented with adhesive side toward the absorbent core and placed on top of the absorbent core. To complete the test assembly, the cover layer was placed over the transfer layer material to be tested.

Another structure according to the invention (Example 4) was also tested using the Fluid Penetration Test. This structure comprised a nonwoven layer with a plurality of disconnected macrofeatures. This structure was made as follows. Both the body-contacting layer and the second layer comprised nonwovens. The body-contacting layer comprised a point bonded nonwoven comprising a blend of 40% 3 denier and 60% 6 denier polypropylene staple fibers with a basis weight of 34 grams per square meter (gsm). The second layer in this example was made from a 30 gsm starting nonwoven comprising a blend of 50% polyester fibers and 50% bicomponent fibers having a sheath of co-polyester around a polyester core, and available from Libeltex n.v. in Meulebeke, Belgium.

Discrete macrofeatures were formed on the appropriate nonwoven layer by heat shaping the starting nonwoven with a metal plate having a regular, repeating pattern of truncated cones. The heat shaping of the starting nonwoven was accomplished by placing the starting nonwoven between the metal plate and a 6.35 mm thick rubber back-up surface and pressing at a pressure of 30.1 kg force per square centimeter and a temperature of 107°C for 15 seconds. The metal plate had a repeating pattern of truncated cones in staggered rows on 6.36 mm centers. Each cone was approximately 3.5 mm in diameter at its base and 1.2 mm in diameter at its top and 2.8 mm high. The heat shaping created discrete macrofeatures on the surface of the nonwoven.

When the body-contacting layer was placed over the second layer with the macrofeatures projecting in the direction of the body-facing layer, the two layers contacted each other substantially only through the macrofeatures in the second layer.

This two-layer structure was placed over an absorbent core material comprising wood pulp and superabsorbent polymer, such as that described in US 5,916,670 to Tan et al., which is incorporated herein by reference. The two-layer structure was placed against the absorbent core material with the second layer facing the absorbent core material. A fluid-impermeable barrier layer was placed on the opposite surface of the absorbent core material to form an absorbent article for use in absorbing body fluids, such as, for example, menstrual fluid.

As a comparison, a two layer structure comprising the same nonwoven layers, but neither layer comprising macrofeatures (Example 4 Control), was also subjected to the Fluid Penetration Test.

Table 1 describes commercial products tested and the absorbent test assemblies made using examples of the present invention and examples representing prior art.

| **Assembly** | **Cover Layer** | **Transfer Layer** | **I Absorbent** | **Barrier** |
|---|---|---|---|---|
| **Commercial Sample 1** | Stayfree Ultra Thin Long with Wing, a commercial product sold in the U.S.A. by Personal Products Company, Inc. | | | |
| **Commercial Sample 2** | Always Ultra Long with Flexi-Wing, a commercial product sold in the U.S.A. by Procter & Gamble, Inc. | | | |
| **Prior Art 1** | Cover Layer¹ | Material of Fig. 24 | Absorbent Core² | Barrier³ |
| **Prior Art 2** | Cover Layer¹ | Material of Fig. 25 | Absorbent Core² | Barrier³ |
| **Example 1** | Cover Layer¹ | Material of Fig. 26 | Absorbent Core² | Barrier³ |
| **Example 2** | Cover Layer¹ | Material of Fig. 2 | Absorbent Core² | Barrier³ |
| **Example 3** | Cover Layer¹ | Material of Fig. 1 | Absorbent Core² | Barrier³ |
| **Example 4**^{**5**} | Cover Layer¹ | 30 gsm Libeltex w Macrofeatures | Absorbent Core⁴ | NA |
| **Ex. 4 Control**^{**5**} | Cover Layer¹ | 30 gsm Libeltex | Absorbent Core⁴ | NA |

| | | | | |
|---|---|---|---|---|
| Note 1: Cover Layer is the Cover Layer of Commercial Sample 1 | | | | |
| Note 2: Absorbent Core is the absorbent core of Commercial Sample 1 | | | | |
| Note 3: Barrier is the Barrier Layer of Commercial Sample 1 | | | | |
| Note 4: Absorbent Core is the absorbent core of of Example 4 | | | | |
| Note 5: Barrier Layer eliminated; all layers cut to 5.1 cm by 10.2 cm. | | | | |

It has been found that structures of the present invention comprising three-dimensional apertured films or nonwovens with a plurality of disconnected macrofeatures have improved fluid handling properties. In particular, the structures had a low Fluid Penetration Time when used as a component layer in disposable absorbent products. Additionally, the structures comprising three-dimensional apertured films exhibited a Repeat Insult Rate that increases less than about 40% over six insults.

Fluid Penetration Time and Repeat Insult Time are measured according to the following test methods, respectively. Testing was performed in a location conditioned to 21 degrees centigrade and 65% relative humidity. Prior to performing the tests, the commercial samples and test assemblies were conditioned at for at least 8 hours.

Fluid Penetration Time (FPT) is measured by placing a sample to be tested under a Fluid Penetration Test orifice plate. The orifice plate consists of a 7.6 cm X 25.4 cm plate of 1.3 cm thick polycarbonate with an elliptical orifice in its center. The elliptical orifice measures 3.8 cm along its major axis and 1.9 cm along its minor axis. The orifice plate is centered on the sample to be tested. A graduated 10 cc syringe containing 7 ml of test fluid is held over the orifice plate such that the exit of the syringe is approximately 3 inches above the orifice. The syringe is held horizontally, parallel to the surface of the test plate, the fluid is then expelled from the syringe at a rate that allows the fluid to flow in a stream vertical to the test plate into the orifice and a stop watch is started when the fluid first touches the sample to be tested. The stop watch is stopped when the surface of the sample first becomes visible within the orifice. The elapsed time on the stop watch is the Fluid Penetration Time. The average Fluid Penetration Time(FPT) is calculated from the results of testing five samples.

| | |
|---|---|
| PRIOR ART 1 | 82.6 |
| EXAMPLE 1 | 59.3 |
| | |
| PRIOR ART 2 | 62.3 |
| EXAMPLE 2 | 42.2 |
| | |
| EXAMPLE 4 | 13.6 |
| EXAMPLE 4 | |
| CONTROL | 106.6 |

The Repeat Insult Time is measured by placing a sample to be tested on a Resilient Cushion, covering the sample with a Repeat Insult Orifice Plate, then applying test fluid according to the schedule described.

The Resilient Cushion is made as follows: a nonwoven fabric of low density (0.03-0.0 g/cm3, measured at 0.24 kPa or 0.035 psi) is used as a resilient material. The nonwoven fabric is cut into rectangular sheets (32×14cm) which are placed one on top of another until a stack with a free height of about 5 cm. is reached. The nonwoven fabric stack is then wrapped with one layer of 0.01 mm thick polyurethane elastomeric film such as TUFTANE film (manufactured by Lord Corp., UK) which is sealed on the back with double-face clear tape.

The Repeat Insult orifice plate consists of a 7.6 cm X 25.4 cm plate of 1.3 cm thick polycarbonate with a circular orifice in its center. The diameter of the circular orifice is 2.0 cm. The orifice plate is centered on the sample to be tested. A graduated 10 cc syringe containing 2 ml of test fluid is held over the orifice plate such that the exit of the syringe is approximately 1 inch above the orifice. The syringe is held horizontally, parallel to the surface of the test plate, the fluid is then expelled from the syringe at a rate that allows the fluid to flow in a stream vertical to the test plate into the orifice and a stop watch is started when the test fluid first touches the sample to be tested. The stop watch is stopped when the surface of the sample first becomes visible within the orifice. The elapsed time on the stop watch is the first fluid penetration time. After an interval of 5 minutes elapsed time, a second 2 ml of test fluid is expelled from the syringe into the circular orifice of the Repeat Insult Orifice Plate and timed as previously described to obtain a second fluid penetration time. This sequence is repeated until a total of six fluid insults, each separated by 5 minutes, have been timed. The Percent Increase in Fluid Penetration Time after Six Insults is calculated as: 100 times the difference between the first and sixth insult times divided by the first insult time. The Average Percent Increase in Fluid Penetration Time is calculated from the results of testing five samples.

**TABLE 3**

| **REPEAT INSULT TIME** | | | | | | | | |
|---|---|---|---|---|---|---|---|---|
| **SAMPLE** | **INSULT # (time in seconds)** | | | | | | **DIFFERENCE in seconds** | **%** |
| | **1** | **2** | **3** | **4** | **5** | **6** | **between Insults 6 & 1** | **INCREASE** |
| COMMERCIAL | | | | | | | | |
| SAMPLE 1 | 5.3 | 7.3 | 12.1 | 12.4 | 14.4 | 15.6 | 10.3 | 194.3 |
| COMMERCIAL | | | | | | | | |
| SAMPLE2 | 4.9 | 9.2 | 9.8 | 10.2 | 10.7 | 11.5 | 6.6 | 134.7 |
| PRIOR ART 2 | 13.7 | 16.5 | 21.1 | 22.6 | 24.2 | 23.9 | 10.2 | 74.5 |
| EXAMPLE2 | 10.1 | 8.6 | 9.9 | 10.4 | 11.0 | 11.3 | 1.2 | 11.9 |
| EXAMPLE 3 | 6.7 | 6.1 | 6.4 | 6.6 | 7.0 | 7.0 | 0.3 | 4.5 |

## Claims

1. A two layer structure for use in absorbent articles, comprising:
a fluid permeable first layer;
a fluid permeable second layer in fluid communication with said first layer, said second layer having a substantially planar surface and a first plurality of disconnected macrofeatures extending from said planar surface;
wherein said first layer and said second layer are structured and arranged such that said first layer contacts said second layer at each of said macrofeatures and said first layer contacts said substantially planar surface of said second layer at selected areas located between said macrofeatures.

2. The structure of claim 1, wherein in an area defined within each of said plurality of macrofeatures said first layer is spaced from said second layer.

3. The structure of claim 1 or claim 2, further comprising a second plurality of macrofeatures, said first plurality of macrofeatures and said second plurality of macrofeatures cooperating to produce a plurality of visual design elements.

4. The structure of any one of claims 1 to 3, wherein said second layer is an apertured film.

5. The structure of any one of claims 1 to 4, wherein said first layer is a nonwoven fabric.

6. An absorbent article comprising a two layer structure of any one of claims 1 to 5 overlying an absorbent layer.
